# EUROPEAN PATENT APPLICATION

(11) **EP 4 785 903 A1**
(43) Date of publication of application: **05.08.2026**
(21) Application number: 25305142.9
(22) Date of filing: 31.01.2025
(51) Int. Cl.: A61F 2/00

(54) **HERNIA MESH WITH INTEGRATED FIXATION MADE FROM BARBED YARNS**

(71) Applicant: Sofradim Production, 01600 Trevoux (FR)
(72) Inventor: SIMONS, Damien, 01600 TREVOUX (FR); COUDERC, Xavier, 01600 TREVOUX (FR); MIRA, Anthony, 01600 TREVOUX (FR); FUMET, Nicolas, 01600 TREVOUX (FR)
(74) Representative: Germain Maureau

(57) **Abstract**

A surgical mesh (100) including a reinforcement component (110), one or more fixation components (120) for providing improved adhesion between the surgical mesh and tissue for tissue repair damage, and a securing component (130) for attaching the fixation component to at least one surface of the reinforcement component is disclosed. Fixation component includes one or more surface structures (123) extending out from an elongated body and that increase friction coefficient with the tissue surface thereby reducing and preventing migration of surgical mesh. The surgical mesh may be removed and repositioned without substantially losing its adhesive properties and is suitable for treatment of hernia.

## Description

### FIELD

The present disclosure generally relates to medical devices for the treatment of tissue disorders, and more particularly to a surgical mesh integrated with surface fixation component that may further facilitate the positioning and fixation of the implant to the tissue.

### BACKGROUND

The abdominal wall in humans is composed of fat and muscles interconnected by fascias. When the abdominal wall becomes damaged, a break in continuity occurs in the fascias, allowing part of the peritoneum to slip through and form a sac, or a hernia, containing either fat or part of the intestines. Hernias or incisional hernias (a hernia occurring through a parietal surgical scar), which manifests in the form of a bulge at the surface of the skin imparts pain to the patient and may lead to further complications such as a bowel obstruction leading to more serious injuries including intestine rupture and sepsis. In order to repair or treat the injured area, surgeons may fit a prosthesis in place to prevent additional bulges from forming and initiate skin growth around the weakened anatomical tissue and close the incision.

These prostheses are often made of polymeric materials and are provided as a knitted textile or a mesh structure. Knitting methods allow obtaining a knitted structure having open-worked faces or pores that promote tissue ingrowth after implantation while maintaining the structural integrity of the prosthesis. Once implanted, the mesh must be positioned in the desired location and fixed to the surrounding biological tissues, such as for example the abdominal wall. While these polymeric implants exhibit a small degree of natural initial adhesion with tissue, lack of resistance or friction at the tissue-implant interface results in migration or in a more serious case, detachment of the implant from the tissue area.

Accordingly, current state-of-the art implant utilizes various securing means for fixing the mesh to the damaged area, such as tacks, staples, or sutures. However, these modes of attachment may introduce the potential for human error, may not be easily repositioned, and may require additional procedures to remove the fixing means post-operation.

Given the foregoing, there is a continuous need for devices and methods to provide an effective fixation of implants to the damaged tissue area that are less difficult to implement, are repositionable in tight tissue space, are biocompatible and bioabsorbable, and most preferably, are repositionable without substantially losing adhesive properties. Therefore, there exists a need to provide a solution in which a surgical mesh is coupled with a biologically compatible fixation component that can positioned (or repositioned) in the desired place.

### SUMMARY

In one aspect, the disclosed technology relates to a surgical mesh comprising an elongated body with one or more surface structure on or extending out from the elongated body; a securing component for attaching the fixation component to at least one surface of the reinforcement component. In some embodiments, the reinforcement component comprises biocompatible material, bioabsorbable materials, non-bioabsorbable materials, or any combination thereof. In some embodiments, wherein the reinforcement component comprises a knit structure, woven structure, non-woven structure, or any combination thereof. In some embodiments, the reinforcement component comprises a plurality of pores, wherein the pores have an average diameter ranging from about 0.1 mm to about 5.0 mm.

In some embodiments, the fixation component comprises biocompatible material, bioabsorbable materials, non-bioabsorbable materials, or combinations thereof. In some embodiments, the surface structure comprises a spur, an anchor, a hook, a barb, a grip, a microstructure, or any combination thereof. In some embodiments, the securing component comprises a sewing yarn, an adhesive, or any combination thereof. In some embodiments, the fixation component comprises about 0.1 % coverage to about 80 % coverage on the surface of the reinforcement component, based on a total surface area of the reinforcement component. In some embodiments, the fixation component is attached to the surface of the reinforcement component in a patterned configuration.

In some embodiments, the surgical mesh is cut to a desired size and/or shape to conform to a specific shape and/or size of a target anatomy. In some embodiments, the surgical mesh is folded or rolled, to avoid fixation of the reinforcement component to itself. In some embodiments, the surgical mesh is folded or rolled to avoid fixation of the surgical mesh to a non-target anatomy

In another aspect, the disclosed technology relates to a method for improving fixation of surgical mesh to a tissue, the method comprising: (a) providing a surgical mesh comprising a reinforcement component a fixation component; and (b) securing the fixation component to surface of the reinforcement component, wherein the fixation component comprises an elongated body with one or more surface structure on or extending out from the elongated body configured to grip to tissue. In some embodiments, the surface structure comprises a spur, an anchor, a hook, a barb, a grip, a microstructure, or a combination thereof. In some embodiments, the securing step further comprises sewing the fixation component to the surface of the reinforcement component with a sewing yarn. In some embodiments, the securing step further comprises sewing the fixation component on the surface of the reinforcement component in a patterned configuration.

In another aspect, the disclosed technology relates to a method of treating a patient using a surgical mesh, the method comprising: (a) applying the surgical mesh of any one of clauses 1-12 to damaged tissue area to provide a contact induced adhesion between the tissue area and the surgical mesh; (b) securing the fixation component to secure the surgical mesh with the damaged tissue area; and (c) promoting tissue repair through and around the surgical mesh. In some embodiments, the method further comprises cutting the surgical mesh to a desired size or shape before the applying in step (a) to conform to a specific shape or size of the damaged tissue area. In some embodiments, the applying in step (a) further comprises rolling or folding the surgical mesh and inserting the surgical mesh to the damaged tissue area. In some embodiments, the applying in step (a) further comprises rolling or folding the surgical mesh and inserting the surgical mesh to the damaged tissue area. In some embodiments, the securing in step (b) further comprises removal and repositioning of the surgical mesh without substantial reduction in adhesion strength.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 depicts a perspective view of a first embodiment of a surgical mesh with a fixation component secured to a reinforcement component.
FIG. 2A depicts a perspective view of a fixation component according to an embodiment of the present disclosure.
FIG. 2B depicts a perspective view of a fixation component according to another embodiment of the present disclosure.
FIG. 2C depicts a perspective view of a fixation component according to another embodiment of the present disclosure.
FIG. 3A depicts a perspective view of a fixation component according to another embodiment of the present disclosure.
FIG. 3B depicts a perspective view of a fixation component according to another embodiment of the present disclosure.
FIG. 4 depicts an enlarged perspective view of a surgical mesh with a fixation component secured to a reinforcement component.
FIG. 5 depicts a perspective view of a second embodiment of a surgical mesh with a fixation component secured to a reinforcement component.
FIG. 6 depicts a perspective view of a third embodiment of a surgical mesh with a fixation component secured to a reinforcement component.
FIG. 7 depicts a perspective view of a fourth embodiment of a surgical mesh with a fixation component secured to a reinforcement component.
FIG. 8 depicts a perspective view of a fifth embodiment of a surgical mesh with a fixation component secured to a reinforcement component.
FIG. 9 depicts a perspective view of a sixth embodiment of a surgical mesh with a fixation component secured to a reinforcement component.
FIG. 10 depicts a perspective view of a seventh embodiment of a surgical mesh with a fixation component secured to a reinforcement component.
FIG. 11 depicts a perspective view of an eighth embodiment of a surgical mesh with a fixation component secured to a reinforcement component.
FIG. 12 depicts perspective view of a ninth embodiment of a surgical mesh with a fixation component secured to a reinforcement component.
FIG. 13A depicts a cross-section view of a surgical mesh in a folded configuration.
FIG. 13B depicts a cross-section view of a surgical mesh in a rolled configuration.
FIG. 14A depicts a perspective view of another embodiment of a surgical mesh with a fixation component secured to a reinforcement component according to an example of the present disclosure.
FIG. 14B depicts a perspective view of another embodiment of a surgical mesh with a fixation component secured to a reinforcement component according to an example of the present disclosure.
FIG. 15 depicts a graph representing the adhesion strength of the surgical mesh of the example of the present disclosure.

### DETAILED DESCRIPTION

Embodiments of the present disclosure relate generally, for example, to systems for treating tissue disorders, and more particularly, to surgical mesh used to treat or alleviate tissue damage. Embodiments of the devices and methods are described below and with reference to the Figures.

The following discussion omits or only briefly describes certain components, features and functionality related to medical implants, installation tools, and associated surgical techniques, which are apparent to those of ordinary skill in the art. It is noted that various embodiments are described in detail with reference to the drawings, in which like reference numerals represent like parts and assemblies throughout the several views, where possible. Reference to various embodiments does not limit the scope of the claims appended hereto because the embodiments are examples of the inventive concepts described herein. Additionally, any example(s) set forth in this specification are intended to be non-limiting and set forth some of the many possible embodiments applicable to the appended claims. Further, particular features described herein can be used in combination with other described features in each of the various possible combinations and permutations unless the context or other statements clearly indicate otherwise.

Terms such as "same," "equal," "planar," "coplanar," "parallel," "perpendicular," etc. as used herein are intended to encompass a meaning of exactly the same while also including variations that may occur, for example, due to manufacturing processes and tolerances. The term "substantially" may be used herein to emphasize this meaning, particularly when the described embodiment has the same or nearly the same functionality or characteristic, unless the context or other statements clearly indicate otherwise. Additionally, it shall be understood that the term "about" encompasses a variation of at least +/- 10% from the example values provided herein.

The following discussion includes a description of a reinforcement component integrated with a fixation component for improved adhesion to damaged tissue area, methods of improving fixation of surgical mesh to a tissue, and related methods of utilizing the surgical mesh to treat tissue-related disorders in accordance with the principles of the present disclosure. The disclosed technology allows for a surgical mesh to incorporate both the reinforcement component and the fixation component allowing for facile and more efficient surgery. The fixation component integrated on the reinforcement component allows for immediate adhesion with minimal pressure, and reduces migration of the surgical mesh once placed, and the mesh structure of the reinforcement component allows for tissue ingrowth for stronger tissue fixation and repair.

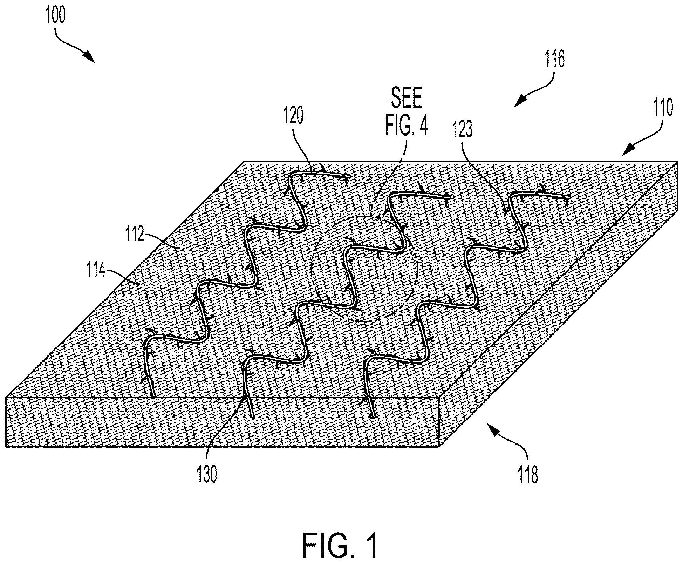

Alternate embodiments are also disclosed. Reference is made in detail to the example embodiments of the present disclosure, which are illustrated in the accompanying figures. FIGS. 1-12, illustrate various components of a surgical mesh, such as, for example, a surgical mesh 100, 400, 500, 600, 700, 800, 900, 1000, 1100a, 1100b, and 1200 having fixation component 120, 420, 520, 620, 720, 820, 920, 1020, 1120a, 1120b and 1220.

Various embodiments and components of the surgical mesh of the present disclosure may be fabricated from any biocompatible material, including non-bioabsorbable materials, biodegradable and/or bioabsorbable materials, and combinations thereof.

As used herein, the term "biocompatible" refers generally to compatibility with living tissue or a living system. As used in this disclosure, various components of the disclosed surgical mesh including the reinforcement component, fixation component, or securing component of the present invention are preferably substantially nontoxic and/or substantially non-injurious to the living tissue or living system. Moreover, such materials preferably do not cause a substantial immunological reaction or rejection in the amounts required over the period of contact or exposure.

As used herein the term "bioabsorbable" or "biodegradable" is understood to mean that the materials having this property are decomposed, or lose structural integrity under body conditions (e.g. enzymatic degradation or hydrolysis) or are broken down (physically or chemically) under physiologic conditions in the body such that the degradation products are excretable or absorbable by the body after a certain time, which may vary, for example, from a few hours to a few years, depending on the chemical nature of the materials. In this disclosure, the term "bioabsorbable" or "biodegradable may be used interchangeably.

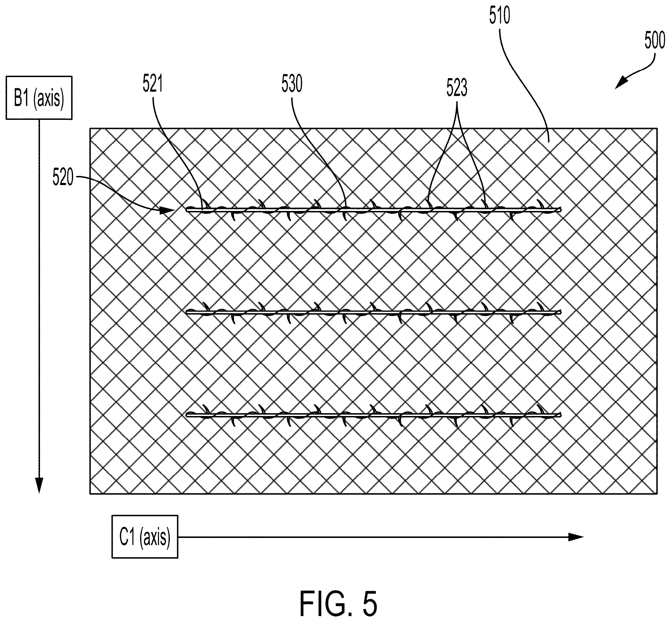

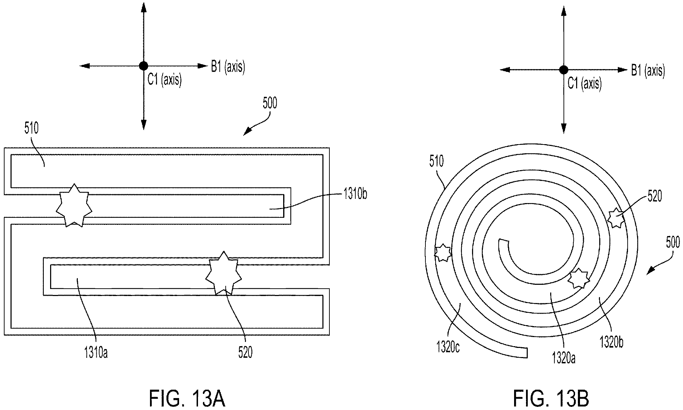

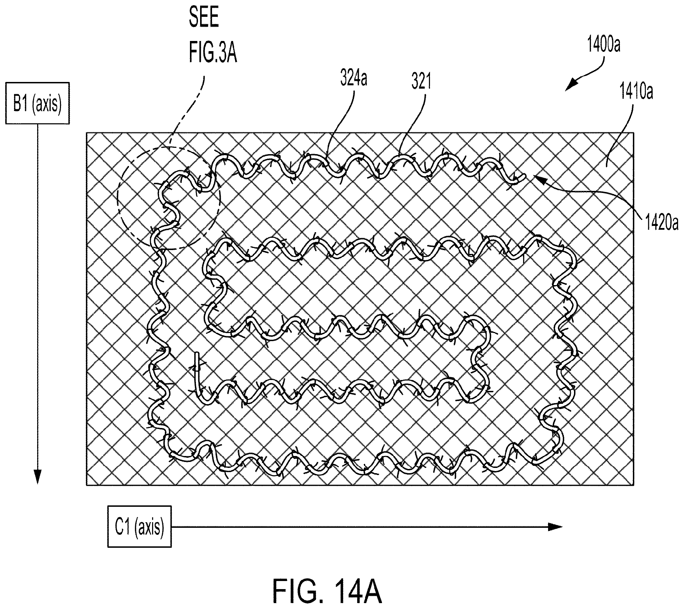

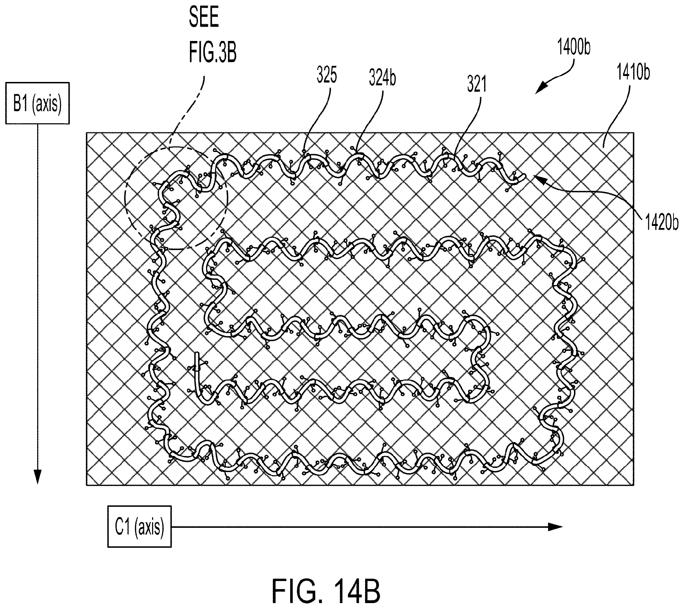

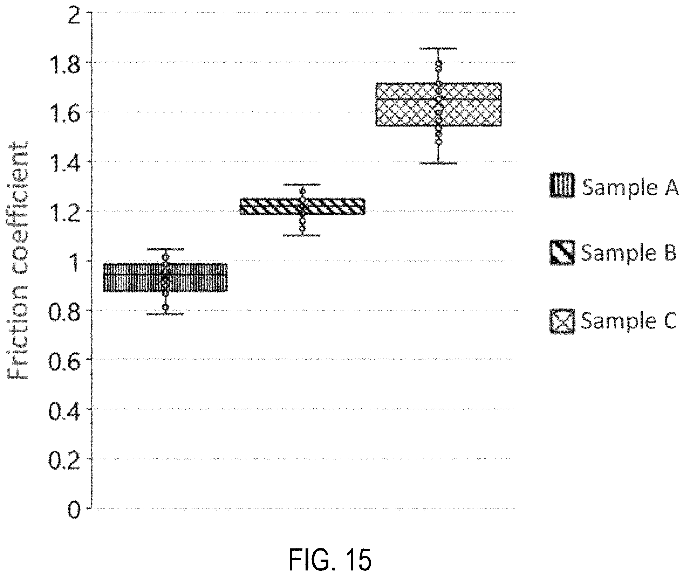

Referring generally to FIGS. 1-4, a surgical mesh comprising a reinforcement component and fixation component according to the present disclosure is shown. FIGS. 5-12 depict various embodiments of a surgical mesh comprising a reinforcement component and fixation component. FIGS 13A and 13B depict various cross sections of surgical mesh in a folded or rolled configuration. FIGS. 14a and 14b depict additional embodiments of a surgical mesh according to the examples of the present disclosure. FIG. 15 depicts the adhesive properties of a surgical mesh comprising the photocurable adhesive according to the present disclosure.

In FIG. 1, a surgical mesh assembly according to an embodiment of the present disclosure, is disclosed. A surgical mesh 100 may comprise reinforcement component 110, one or more fixation component 120 on the surface of the reinforcement component 110 and securing component 130 configured to attach fixation component 120 to the surface of reinforcement component 110. In some embodiments, reinforcement component 110 may be designed with a network of yarns or filaments 112. In some embodiments, reinforcement component 110 may comprise a knit structure, woven structure, non-woven structure, or a combination thereof formed from one or more filaments 112.

As used herein a "knit structure" is understood as an arrangement of biocompatible yarns, such as a textile or fabric in an inter-looping one or more yarns in horizontal rows and vertical columns of stitches. As used herein a "woven structure" is understood as an arrangement of biocompatible yarns, such as a textile or fabric in an inter-lacing two sets of threads. As it is related to the woven structure, two sets of threads, called warp and weft, are interlaced at right angles forming a weave. As used herein a "non-woven structure" is understood as an arrangement of yarns, such as a textile or fabric in a bonded configuration using methods like heat, chemical adhesive, or mechanical entanglement without conventional, weaving or knitting process, producing a random, web-like structure.

In some embodiments, reinforcement component 110 may be formed into any of the aforementioned structures using any suitable method known to those of ordinary skill including, but not limited to, weaving, knitting, braiding, crocheting, embroidering, and the like. In some embodiments, reinforcement component 110 may include a knit mesh body. In some embodiments, the implantable knit mesh may be knitted on a warp knitting machine, of the tricot or Raschel type, with at least two or three guide bars. In some embodiments, the implantable mesh may be made as provided in: U.S. Patent Nos. 6,596,002 to Therin et al.; 7,331,199 to Ory et al.; 9,186,235 to Ory et al.; and 9,510,927 to Simons, the entire contents of each reference are incorporated herein by reference.

In some embodiments, reinforcement component 110 may include a two-dimensional mesh body. For example, reinforcement component 110 may include one or more filaments intertwined in a generally planar or horizontal criss-crossing pattern, i.e., weft/warp pattern, forming a mesh body including a single layer of intertwined filament(s).

In some embodiments, reinforcement component 110 may include a three-dimensional mesh body. For example, the mesh body may include one or more filaments intertwined in both a horizontal and vertical pattern forming a mesh body including two or more layers of intertwined filament(s) often connected to each other by a spacer filament, i.e., a filament that extends between and connects the two or more layers, defining a space or depth between the two or more layers.

As used herein a "mesh" is understood as an arrangement of biocompatible yarns, such as a textile or fabric, preferably open-worked, provided with pores that favor colonization of tissue such as cellular growth. Such a mesh can be bioabsorbable/biodegradable, permanent or partially bioabsorbable/biodegradable. It is sufficiently flexible to be folded up at the time of introduction into the abdominal cavity. Meshes for forming hernia prostheses are well known to a person skilled in the art. The mesh can be supplied in any shape whatsoever, for example generally rectangular, square, circular, oval, and can then be cut to suit the shape of the hernia defect. For example, the overall shape of the mesh can be circular or oval. Alternatively, the mesh can have a generally square shape, a rectangular shape, a diamond shape, or other polygonal shapes including but not limited to pentagon, hexagon, octagon, or in 3-dimensional configuration, i.e., having different topography to fit certain soft tissue areas of corresponding shapes.

In some embodiments, the filaments of the reinforcement component of the surgical mesh may be interwoven in a configuration that provides a plurality of pores 114, thereby forming a mesh or mesh-like structure. In some embodiments, pores 114 of reinforcement component 110 may have an average size or diameter between about 0.1 mm and about 5 mm, about 0.2 mm to about 45 mm, about 0.3 mm to about 40 mm, about 0.4 mm to about 35 mm, or about 0.5 mm to about 3 mm.

In some embodiments, reinforcement component 110, and particularly filament 112 that form the network or mesh may be made from any biocompatible material, including bioabsorbable materials, non-bioabsorbable materials, and combinations thereof. In some embodiments, bioabsorbable materials may include, but are not limited to: polysaccharides, such as alginate, dextran, chitin, hyaluronic acid, cellulose, fucans, glycosaminoglycans, and chemical derivatives thereof (substitutions and/or additions of chemical groups, for example, alkyl, alkylene, hydroxylations, oxidations, and other modifications routinely made by those skilled in the art); and proteins, such as collagen, gelatin, albumin, casein, zein, silk, and copolymers and blends thereof, alone or in combination with synthetic polymers such as but not limited to poly-lactic acid, polyglycolide, Poly-4-hydroxybutyrate, polyethylene terephthalate, polypropylene, and polyamides.

In some embodiments, synthetically modified natural polymers may include, but are not limited to, cellulose derivatives, such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, and cellulose sulfate sodium salt.

In some embodiments, representative synthetic bioabsorbable polymers may include, but are not limited to, polyhydroxy acids prepared from lactone monomers, such as glycolide, lactide, caprolactone, ε-caprolactone, valerolactone, and δ-valerolactone, as well as pluronics, carbonates (e.g., trimethylene carbonate, tetramethylene carbonate, and the like), dioxanones (e.g., 1,4-dioxanone and p-dioxanone), 1,dioxepanones (e.g., 1,4-dioxepan-2-one and 1,5-dioxepan-2-one), and combinations thereof. Polymers formed therefrom include: polylactides; poly(lactic acid); polyglycolides; poly(glycolic acid); poly(trimethylene carbonate); poly(dioxanone); poly(hydroxybutyric acid); poly(hydroxyvaleric acid); poly(lactide-co-(ε-caprolactone-)); poly(glycolide-co-(ε-caprolactone)); polycarbonates; poly(pseudo amino acids); poly(amino acids); poly(hydroxyalkanoate)s; polyalkylene oxalates; polyoxaesters; polyanhydrides; polyortho esters; and copolymers, block copolymers, homopolymers, blends, and combinations thereof.

In some embodiments, non-limiting examples of suitable non-bioabsorbable materials may include, but are not limited to polyolefins, such as polyethylene and polypropylene including atactic, isotactic, syndiotactic, and blends thereof; polyethylene glycols; polyethylene oxides; ultrahigh molecular weight polyethylene; copolymers of polyethylene and polypropylene; polyisobutylene and ethylene-alpha olefin copolymers; fluorinated polyolefins, such as fluoroethylenes, including expanded polytetrafluoroethylene (ePTFE) and condensed polytetrafluoroethylene (PTFE), fluoropropylenes, fluoroPEGSs, and polytetrafluoroethylene; polyamides, such as nylon and polycaprolactam; polyamines; polyimines; polyesters, such as polyethylene terephthalate and polybutylene terephthalate; aliphatic polyesters; polyethers; polyether-esters, such as polybutester; polytetramethylene ether glycol; 1,4-butanediol; polyurethanes; acrylic polymers and copolymers; modacrylics; vinyl halide polymers and copolymers, such as polyvinyl chloride; polyvinyl alcohols; polyvinyl ethers, such as polyvinyl methyl ether; polyvinylidene halides, such as polyvinylidene fluoride and polyvinylidene chloride; polyacrylonitrile; polyaryletherketones; polyvinyl ketones; polyvinyl aromatics, such as polystyrene; polyvinyl esters, such as polyvinyl acetate; copolymers of vinyl monomers with each other and olefins, such as etheylene-methyl methacrylate copolymers, acrylonitrile-styrene copolymers, ABS resins, and ethylene-vinyl acetate copolymers; alkyd resins; polycarbonates; polyoxymethylenes; polyphosphazine; polyimides; epoxy resins; aramids, rayon; rayon-triacetate; spandex; silicones; and combinations thereof.

In some embodiments, reinforcement component 110 of the present disclosure may comprise barbs wound from individual filaments or yarn and may include hook features produced from the aforementioned biocompatible material, producing a Velcro^{®}-like hook and loop configuration such that when surgical mesh 100 comprising reinforcement component 110 is folded on itself, it can be separated reversibly. Suitable mesh structure in accordance with the present technology may include commercially available surgical mesh from the company Sofradim Production under the trade name Parietex^{®} Progrip or Parietene^{®} Progrip. Further details regarding surgical mesh structure are illustrated and described, for example in U.S. Patent Nos. 9,744,019 to Montanari et al. and 10,865,505 to Montanari et al., the entire contents of each disclosure are incorporated herein by reference.

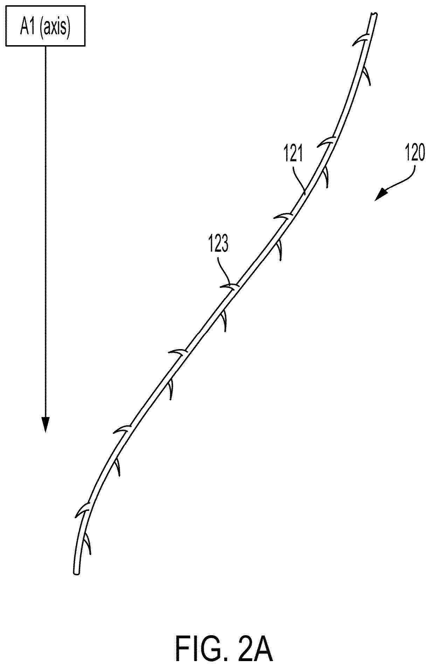

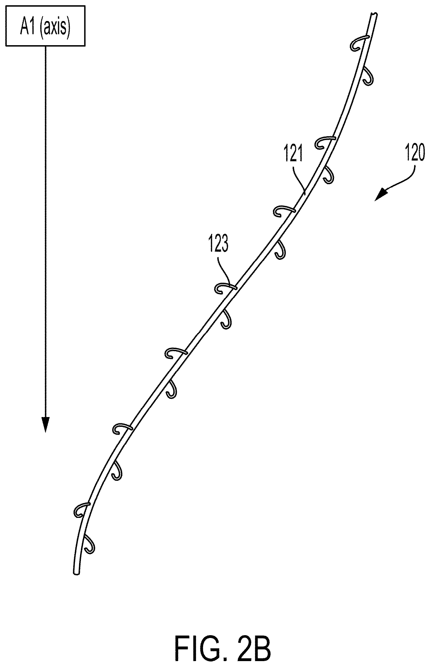

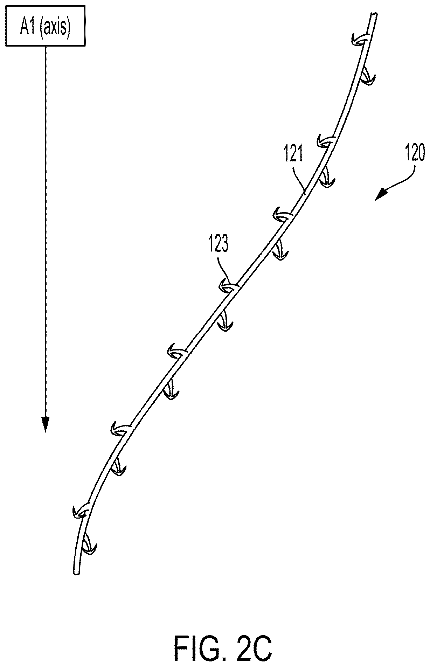

Now referring to FIG. 2, an embodiment of fixation component 120 capable of use with the aspects of the present disclosure is shown. As seen in FIGS. 1 and 2, fixation component 120 is configured to attach to the surface of reinforcement component 110 to provide surgical mesh 100 with a surface or an interface for mechanical fixation of surgical mesh 100 with tissue surface. In some embodiments, fixation component 120 is configured to adhere immediately to biological tissues when in contact with them at low contact pressure, i.e., below about100 N/m², about 90 N/m², about 80 N/m², or about 75 N/m².

In some embodiments, fixation component 120 may generally comprise an elongated body 121 extending from one end to opposite end in an axial direction along axis A1 with one or more surface structure(s) 123 on the surface of or extending out from elongated body 121. In some embodiments, fixation component 120 may be fabricated from any of the biocompatible, bioabsorbable, non-bioabsorbable materials or a combination of materials described above with respect to reinforcement component 110.

As seen in FIG. 1 and seen best in FIGS. 2A-2C, fixation component 120 may comprise a plurality of surface structures 123 configured to provide mechanical fixation, i.e., grip onto patient tissue. When engaged with surface tissue area, surface structures 123 provide a fixation that increases the effective friction at each structure-tissue interface. This increase in friction provides enhanced fixation of surgical mesh 100 to tissue and reduces or prevents migration of surgical mesh 100 post-operation. In some embodiments, surface structure 123 may comprise various protruding features including a spur, an anchor, a hook, a barb, a grip, a microstructure such as micro-bumps, micro-serration, or any combination thereof. For instance, as seen in FIG. 2A, surface structure 123 may be a barb structure. As seen in FIG. 2B, surface structure 123 may be a hook structure. As seen in FIG. 2A, surface structure 123 may be an anchor structure. In some embodiments, fixation component 120 may be coated with an additional adhesive that provides chemical adhesion with biological tissues for additional reinforcement. In some embodiments, fixation component 120 may be coated with additional drugs or other therapeutics that facilitate tissue repair and promote tissue growth.

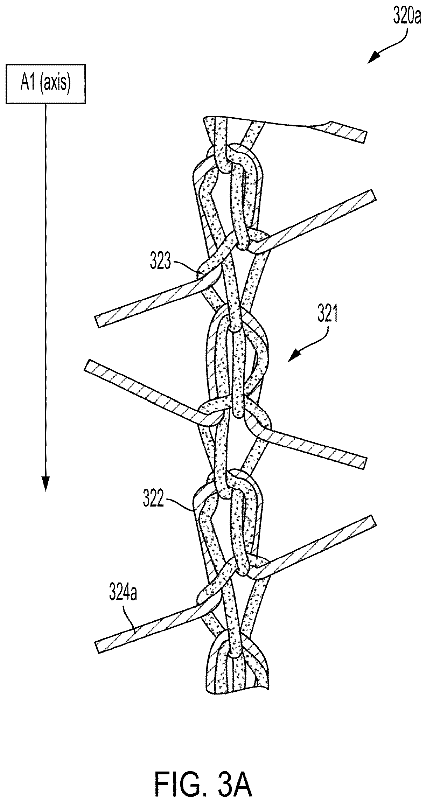

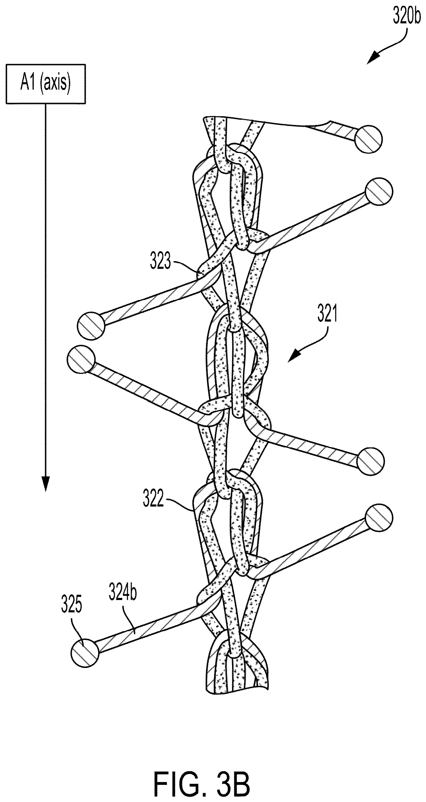

While shown as a monolithic, monofilament thread, in some embodiments, fixation component may be formed from braided threads, multifilament threads, and other suitable surgical fibers and sutures. As seen in FIGS. 3A and 3B, in some embodiments, fixation component 320a (see FIG. 3A) or 320b (see FIG. 3B) may be formed from a plurality of braided threads. Individual threads 322 and 323 may be braided to form body 321 of fixation component 320a or 320b. In some embodiments, body 321 may be braided using a plurality of threads 322 and 323, such that more than one thread 322 and/or thread 323 may be present. In some embodiments, thread 322 and thread 323 may be composed of the same material or different material from each other and they may be selected from any of the materials disclosed above. In some embodiments, one thread or both threads 323 or 322 may be cut during the braiding process to produce surface structures 324a and/or 324b. As seen in FIG. 3A, thread 322 may be cut in a configuration such that surface structure 324a comprises a sharp or jagged tip that provides mechanical fixation/gripping to a damaged tissue area, e.g., a "cut" barb. In some embodiments, as seen in FIG. 3B, surface structure 324b may further comprise additional an gripping feature 325 at the end tip of surface structure 324b, e.g., a "grip" barb. In some embodiments, gripping feature 325 may comprise a spur, an anchor, a hook, a barb, a grip, a microstructure such as micro-bumps, micro-serration, or any combination thereof.

Further details regarding materials and structural information of fixation component 120, 320a and 320b are illustrated and described, for example in: U.S. Patent Nos. 11,098,422 to Bailly et al. and 10,178,991 to Bailly et al., the entire contents of each disclosure are incorporated herein by reference. Suitable fixation components in accordance with the present technology may include commercially available fixation component from the company Sofradim Production under the trade name, T-Loc^{™} wound closure device, or a yarn where the surface structure incorporate materials with adhesion properties, or yarn where the surface is abrased such as created relief which may create a "suction" effect.

In some embodiments, individual surface structure 123 may be arranged in various configurations for example, helical, linear, or randomly spaced. In some embodiments, surface structure 123 may be arranged in symmetrical or asymmetrical configuration along the axis A1 or a cross-section of elongated body 121. In some embodiments, the number, configuration, spacing, and surface area of microstructure 123 may vary depending upon the tissue in which fixation component 120 is utilized, as well as the composition and structure of fixation material. 120. For example, if fixation component 120 is to be used to reinforce a tissue patch for a tendon, microstructure 123 may be made relatively short and more rigid to facilitate gripping to the firmer tissue. Alternatively, if fixation component 120 is intended to be used to reinforce softer tissue than a tendon e.g., a muscle, microstructure 123 may be made longer and spaced further apart to increase the ability of microstructure 123 to grip the soft tissue.

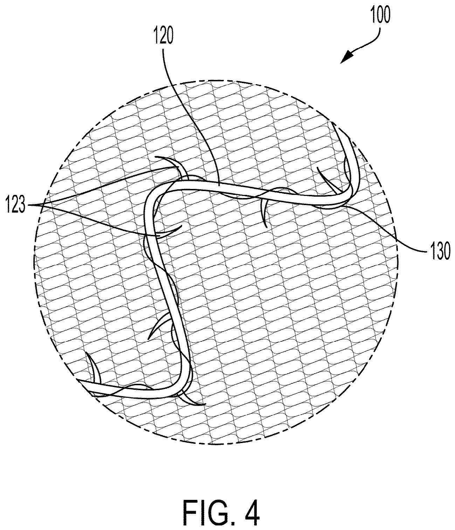

As seen in FIG 1 and seen better in FIG. 4, in some embodiments, surgical mesh 100 may comprise a securing component 130 that is used to attach fixation component 120 to the surface of reinforcement component 110. In some embodiments, securing component 130 may comprise a sewing yarn, an adhesive, or a combination thereof. In some embodiments, securing component 130 may be an adhesive comprising biocompatible, bioabsorbable, or non-bioabsorbable materials. In some embodiments, securing component 130 may be a sewing yarn to be fabricated from any of the biocompatible, bioabsorbable, and non-bioabsorbable materials described above with respect to reinforcement component 110 or fixation component 120. In some embodiments, fixation component 120 may be attached to reinforcement component 110 by methods including: knitting, sewing, and tailored fiber placement technology (TFP). Further details regarding surgical mesh structure are illustrated and described, for example in U.S. Patent No. 11,471,257 to Simons et al., the entire contents of which are incorporated herein by reference. In some embodiments, fixation component 120 may comprise a surface coverage from about 0.1 % to about 80 %, about 5% to about 80%, about 10% to about 80%, about 20% to about 70%, about 30% to about 60%, about 40% to about 50%, about 0.1% to about 5%, about 5% to about 10%, about 10% to about 20%, about 20% to about 30%, about 30% to about 40%, about 40% to about 50%, about 50% to about 60%, about 60% to about 70%, or about 70% to about 80%, about on reinforcement component 110, based on the total surface area of reinforcement component 110. In some embodiments, fixation component 120 may be attached to reinforcement component 110 by sewing fixation component 120 with a securing component 130, e.g., sewing yarn.

In some embodiments, fixation component 120 may be attached to reinforcement component 110 by sewing fixation component 120 with a securing component 130 on the top surface 116, the bottom surface 118, or both the top surface and the bottom surface as described in FIG. 1. In this case, fixation component 120 may have a surface area coverage at each surface from about 0.1 % to about 80 %, about 5% to about 80%, about 10% to about 80%, about 20% to about 70%, about 30% to about 60%, about 40% to about 50%, about 0.1% to about 5%, about 5% to about 10%, about 10% to about 20%, about 20% to about 30%, about 30% to about 40%, about 40% to about 50%, about 50% to about 60%, about 60% to about 70%, or about 70% to about 80%, based on the total surface area of reinforcement component 110. This allows for selectively adjusting the fixation performance of surgical mesh 100 based on the particular surgical need of a patient.

Now referring to FIGS. 5-12, embodiments of surgical mesh 500, 600, 700, 800, 900, 1000, 1100, and 1200 (i.e., surgical mesh embodiments 500 to 1200) are disclosed. Surgical mesh 500 to 1200 (FIGS. 5-12) may have the same, similar, and/or substantially the same features and functionality, as well as be fabricated using the same, similar and/or substantially the same method as explained above with respect to surgical mesh 100. It should be understood that parts or components with similar numbering, i.e., 110 and 510, 130 and 530, 123 and 523, describe the same or substantially the same features but in a different embodiment, e.g., 100 and 500. For example, surgical mesh 500 may comprise a biocompatible reinforcement component, e.g., 210 fabricated from yarns or filaments, e.g., 212, and a plurality of pores, e.g., 214.

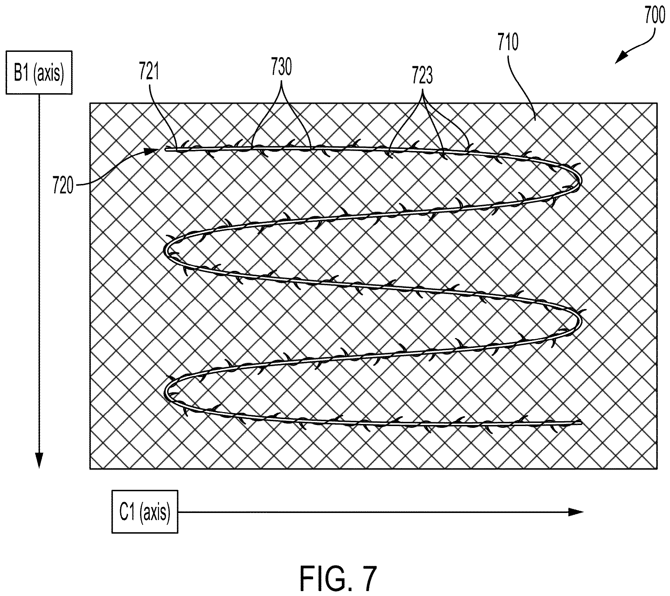

Surgical mesh 500 to 1200 may comprise one or more fixation component(s), e.g., 520, arranged in any patterned configuration including but not limited to: linear, spiral, criss-cross, zigzag, as well as random configurations and custom combinations based on the surgical need of a patient, e.g., a spiral wave configuration. In some embodiments, the fixation component may be arranged in a localized configuration or "zones," i.e., a recto (front) side of the mesh or the back side, in a continuous pattern or a discontinuous pattern) This tunable placement of one or more fixation component(s) provides numerous advantages including cutting the mesh to a desired size and shape such as, but not limited to cutting the mesh around the fixation component(s) to provide fixation of the surgical mesh focused at or in the proximity of the targeted tissue area. This allows the surgical mesh to, for example, potentially avoid key non-targeted anatomy or tissues, e.g., spermatic cord, nerves, and intestines. For example, surgical mesh 700 (as shown in FIG. 7 and further described below) including for example fixation components 720 arranged in a "back-and-forth" pattern may be cut along the shape or an outline of fixation component 720 to administer the surgical mesh to a targeted tissue area to, for example, avoid sensitive and delicate organs, tissues, or nerves.

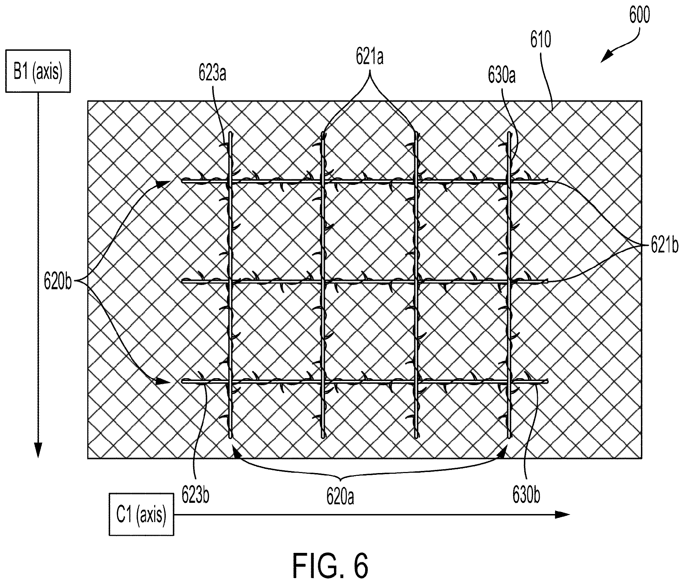

As seen in FIG. 5, surgical mesh 500 may comprise fixation component 520 arranged on a substantially straight or linear type configuration along a lengthwise and widthwise directions of reinforcement component 510. As seen in FIG. 5, surgical mesh 500 may comprise more than one fixation component 520 comprising a plurality of surface structures 523 attached to reinforcement component 510 using securing component 530 in a non-overlapping configuration. In some embodiments, fixation component may be placed from about 0.05 cm to about 50 cm, about 0.5 cm to about 50 cm, about 1 cm to about 50cm, about 1 cm to about 5 cm, about 5 cm to about 10 cm, about 10 cm to about 20 cm, about 20 cm to about 30 cm, about 30 cm to about 40 cm, or about 40 cm to about 50 cm from each other, i.e., they may be sequentially spaced within the stated range. In some embodiments, as seen in FIG. 6, reinforcement component 610 may comprise a first plurality of fixation components 620a along a first direction of reinforcement component 610 and a second plurality of fixation components 620b along a second direction, i.e., forming a criss-cross configuration, of reinforcement component 610 in an overlapping configuration. In this configuration, a first plurality of microstructures 623a on a first plurality of fixation components 620a may engage with a second plurality of microstructures 623b on a second plurality of fixation components 620b to further improve the mechanical/adhesive properties of surgical mesh 600.

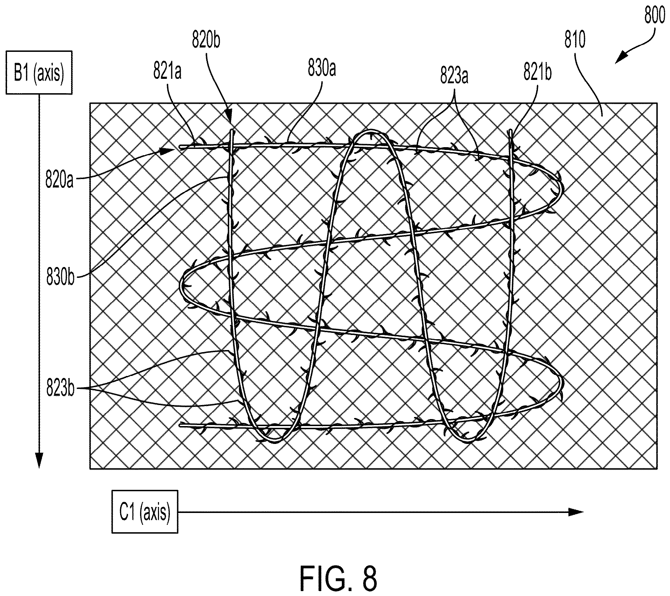

Now referring to FIG. 7, a single fixation component 720 comprising a plurality of surface structures 723 may be arranged along the surface of reinforcement component 710 in a "back-and-forth" "undulating" "wave-like" or "snake-like" configuration. In some embodiments as seen in FIG. 8, fixation components 820a and 820b each comprising body 821a and 821b, surface plurality of surface structures 823a and 823b may be independently arranged in an overlapping configuration and secured using securing components 830a and 830b, to further improve the mechanical/gripping properties of surgical mesh 800.

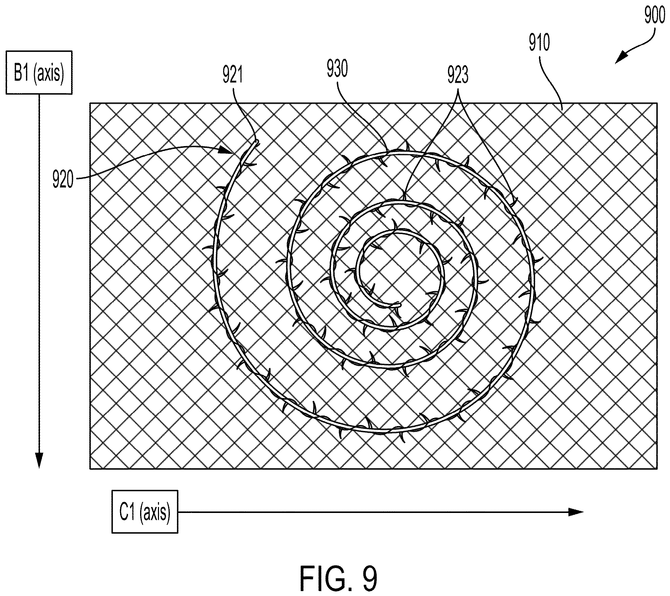

Now referring to FIG. 9, in some embodiments, surgical mesh 900 may comprise fixation component 920 comprising body 921 with plurality of surface structures 923, attached to reinforcement component 910 in a spiral configuration or helical type configuration extending outward from the interior surface to provide optimal reinforcement with tissue contact area at the interior area of surgical mesh 900. Although not shown in FIG. 9, surgical mesh 900 may comprise a plurality of fixation component 920 in a spiral configuration and/or overlapping spiral configurations at localized areas that may experience increased stress, strain, and/or high wear to improve the mechanical/gripping properties of surgical mesh 900.

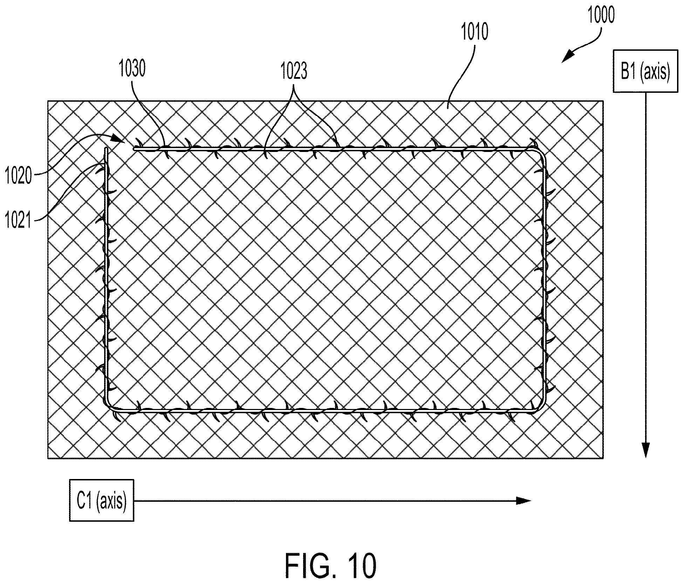

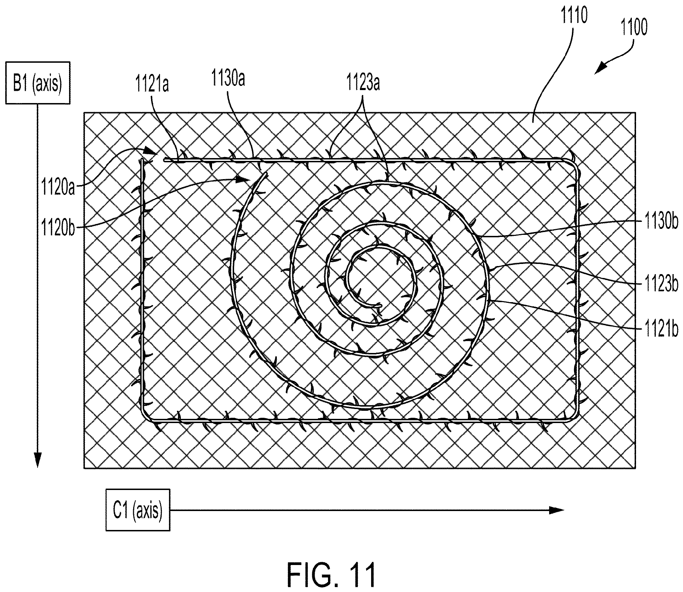

Now referring to FIG. 10, in some embodiments, surgical mesh 1000 may comprise fixation component 1020 comprising body 1021 with plurality of surface structures 1023 may be attached around a perimeter of surgical mesh 1000 to provide optimal reinforcement with the tissue contact area at the edges of surgical mesh 1000. As seen in FIG. 11, in some embodiments, surgical mesh 1100 may comprise first fixation component 1120a and second fixation component 1120b each having a plurality of surface structures 1123a and 1123b, each secured using securing component 1130a and 1130b, arranged at the perimeter and interior, respectively having different, similar, or substantially the same patterns as described above.

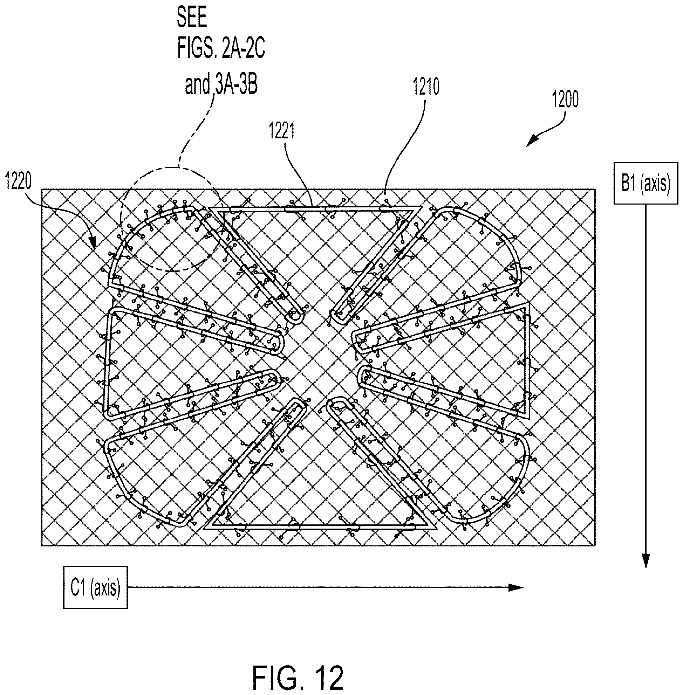

Now referring to FIG. 12, in some embodiments, surgical mesh 1200 may comprise fixation component 1220 comprising body 1221 with plurality of surface structures (e.g., 123 as seen in FIGS. 2A to 2C or 324a and 324b as seen in FIGS. 3A and 3B) attached to reinforcement component 1210 in a patterned configuration. As discussed above, patterned fixation component 1220 on reinforcement component 1210 provides visual and physical indication (e.g., when positioned on damaged tissue area) to help surgeon to center, position, and reposition surgical mesh 1200 surface to the defect/damaged area. It should be understood that additional designs and patterned configurations of fixation component 1220 on reinforcement component 1210 not explicitly disclosed in this document may be considered, based on the surgical need of a patient, including, the location and dimension of the damaged tissue area, presence of sensitive tissue, organs, or bones in the proximity of the damaged area, or surgical limitations such as using a trocar or other surgical devices to introduce the surgical mesh to targeted area.

As indicated in FIG. 12, in some embodiments, fixation component 1220 may be any one of fixation components disclosed in this document including for example, any one of fixation component 120 as seen in FIGS. 2A-2C or fixation components 320a and 320b as seen in FIGS. 3A and 3B, respectively. In some embodiments, fixation component 1220 may be attached to reinforcement component 1210 by any means described above including but not limited to sewing any one of fixation component 1220 with any one of securing component described in this document , for example, using securing component 130.

In some embodiments, fixation component 1220 may comprise the same, similar, and/or substantially the same features and functionality, as well as be fabricated using the same, similar and/or substantially the same method as explained above with respect to fixation component 120 (see FIGS. 2A-2C), that is, fixation component 1220 may comprise an elongated body 1221 that is the same, similar, and/or substantially the same as elongated body 121 (as seen in FIGS. 2A-2C). In this configuration, fixation component 1220 may comprise elongated body 1221 extending from one end to opposite end in an axial direction along axis A1 with one or more surface structure(s) (e.g., 123 of FIGS. 2A-2C) on the surface of or extending out from elongated body 1221 that is capable of attaching to damage tissue area.

In some embodiments, fixation component 1220 may comprise the same, similar, and/or substantially the same features and functionality, as well as be fabricated using the same, similar and/or substantially the same method as explained above with respect to fixation components 320a or 320b (see FIGS. 3A and 3B), that is, fixation component 1220 may be formed from a plurality of braided threads (comprising individual threads e.g., 322 and 323 as seen in FIGS. 3A and 3B). In this configuration, fixation component 1120 may comprise elongated body 1221 extending from one end to opposite end in an axial direction along axis A1 with one or more surface structure(s) (e.g., 324a and 324b of FIGS. 3A and 3B) on the surface of or extending out from elongated body 1221 that is capable of attaching to damage tissue area.

In some embodiments, one or more fixation component(s), e.g., 520, 620a, 620b, 720, 820a, 820b, 920, 1020, 1120a, 1120b, and 1220 may be arranged in any patterned configuration such that mesh-to-mesh fixation, i.e., reinforcement component folding on it-self and adhering to each other, fixation component-to-fixation component adhesion, or mesh-to-fixation component adhesion may be avoided when the surgical mesh is rolled or folded along a width, length, or along any diagonal axis of the surgical mesh during laparoscopic procedure using a trocar or other surgical devices to introduce the surgical mesh to targeted area.

In some embodiments, when the surgical mesh is folded, the fixation component(s) may be arranged on the surface of the reinforcement component such that the folded surfaces of the reinforcement component may not directly oppose each other due to a gap provided by the fixation component(s). For example, as seen in FIG. 13A, surgical mesh 500 may be folded along axis B1, i.e., folded along an imaginary line that is parallel to axis C1 and perpendicular to axis B1, such that each fixation component 520 may not directly come in contact with each other in a folded configuration. Moreover, in this configuration, fixation between the adjacent layers of reinforcement component 510 may also be avoided as a result of folded surface layers 1310a and 1310b providing a gap between the adjacent layers of the reinforcement component 510. In some embodiments, the surgical mesh may be folded once, twice, thrice, or any number of times depending on the structural and spatial limitations of the surgical procedure. For example, as illustrated in FIG. 13A, surgical mesh 500 may be folded twice, forming two folded surface layers 1310a and 1310b where a fixation component 520 is positioned. In some embodiments, if the surgical procedure requires insertion of the surgical mesh to a smaller space or a tighter cavity, the surgical mesh may be additionally folded to further reduce the dimensions, e.g., length or width, of the surgical mesh.

In some embodiments, any of the surgical mesh of the present disclosure may be rolled. Referring to FIG. 13B, surgical mesh 500 may be rolled along axis C1 to form a cross-section along axis C1 direction comprising rolled surface layers 1320a, 1320b, 1320c, each embedded with fixation component 520. In this configuration, fixation component 520 may not directly come in contact with each other in a rolled configuration, thereby potentially avoiding adhesion between fixation component(s) 520. Moreover, in this configuration, fixation between the adjacent layers of reinforcement component 510 may also be avoided as a result of rolled surface layers 1320a, 1320b, and 1320c providing a gap between the adjacent layers of the reinforcement component 510. In some embodiments, the surgical mesh may be rolled with varying tightness, i.e., how many cross-sectional "spiral" is formed, depending on the structural and spatial limitations of the surgical procedure. In some embodiments, a rolled surface layer may comprise zero, or more than one fixation component in each layer. In some embodiments, if the surgical procedure requires insertion of the surgical mesh to a smaller space or a tighter cavity, the surgical mesh may be additionally rolled more tightly, i.e., contains more rolled surface layers, to further reduce the dimensions. In some embodiments, rolled surgical mesh 500 may be inserted into the surgical area using a trocar and opened to apply fixation component(s) 520 to a specific target anatomy.

A related aspect of the present disclosure provides a method for improving fixation of surgical mesh of the present disclosure to a tissue. In some embodiments, a surgeon may provide any surgical mesh of the present disclosure, i.e., 100, 500, 600, 700, 800, 900, 1000, 1100, or 1200, comprising a reinforcement component, i.e., 110, 510, 610, 710, 810, 910, 1010, 1110, or 1210 to a fixation component 520, 620a, 620b, 720, 820a, 820b, 920, 1020, 1120a, 1120b, or 1210 in a various pattern as described above. In some embodiments, the fixation component comprises various surface structures described above including a spur, an anchor, a hook, a barb, a grip, a microstructure, or a combination thereof., may be attached to reinforcement component by various methods disclosed above including but not limited to knitting, sewing, and tailored fiber placement technology (TFP). In some embodiments, the fixation component may be secured to the reinforcement component with a securing component, i.e., sewing yarn. By providing the surgical mesh with one or more fixation component, the friction coefficient, i.e., the effective ratio of the friction force acting between two surfaces in contact to a force pressing the surfaces together, is increased. In other words, a higher friction coefficient (also referred to as a coefficient of friction) indicates greater resistance present between the surgical mesh and the tissue, reducing the risk of surgical mesh migration or detachment.

Another aspect of the present disclosure provides a method of treating a patient using a surgical mesh of the present disclosure. In some embodiments, a surgeon may apply any surgical mesh of the present disclosure, i.e., surgical mesh 100 or 500 to 1200 to the damaged tissue area. By compressing the surgical mesh to the targeted tissue area, a contact-induced adhesion between the tissue and the surgical mesh is achieved. In some embodiments, the surgeon may remove and reposition surgical mesh as needed despite the adhesion that may be present or have occurred. In some embodiments, the surgical mesh may be folded or rolled according to the present disclosure in order to accommodate the size and shape associated with the insertion or application of the surgical mesh to a specific targeted area of varying size, shape, contour, and spatial limitations.

In some embodiments, a fixation component present in any of surgical mesh 100 and 500 to 1200 may provide an increased friction coefficient up to about 90%, about 80%, about 70%, about 60%, about 50%. Afterward, tissue is grown around the exterior edges of the surgical mesh and through the pores of the mesh during the healing process.

### EXAMPLES

The disclosed technology is next described by means of the following examples. The use of these and other examples anywhere in the specification is illustrative only, and in no way limits the scope and meaning of the invention or of any exemplified form. Likewise, the invention is not limited to any particular or preferred embodiments described herein. Indeed, modifications and variations of the invention may be apparent to those skilled in the art upon reading this specification and can be made without departing from its spirit and scope. The invention is therefore to be limited only by the terms of the claims, along with the full scope of equivalents to which the claims are entitled. Efforts have been made to ensure accuracy with respect to values presented (e.g., amounts, temperature, etc.), but some experimental error and deviation should be accounted for.

### Example 1: Fixation properties of various surgical mesh with and without fixation component

Fixation properties of two surgical meshes 1400a and 1400b (as seen in FIGS 14A and 14B) with a fixation component 1420a and 1420b secured on the reinforcement components 1410a and 1410b were the fabricated and compared to a bare surgical mesh as set forth in Table 1. In some embodiments, fixation components 1420a and 1420b may be the same structure as fixation components 320a and 320b, including all of the features (e.g., 321, 323, 322, 324a, 324b, 325) as illustrated in FIGS. 14A and 14B, and described in detail above. In some embodiments, fixation components 320a and 320b may be attached to reinforcement components 1310a and 1310b using any of the attaching methods described above, including but not limited to securing component (e.g., 130, 530, 630a, 630b, etc.), a sewing yarn, an adhesive, or a combination thereof.

As described in this disclosure, surgical mesh 1400a and 1400b may have the same, similar, and/or substantially the same features and functionality, as well as be fabricated using the same similar and/or substantially the same method as explained above with respect to surgical mesh 100 or 500-1200. It should be understood that parts with similar numbering, i.e., 110 and 1310a/1310b, 120 and 1320a/1320b describe the same or substantially the same features.

**Table 1.**

| **Samples** | **Surface Structure** | **Patterned Configuration** | **Surface Coverage of Fixation component** |
|---|---|---|---|
| **A:** Bare polypropylene | N/A | N/A | 0% |
| **B:** Concept 1 (FIG. 13A) | Cut barb (FIG. 3A) | Spiral wave (FIG. 14A) | 1% |
| **C:** Concept 2 (FIG. 13B) | Grip barb (FIG 3B) | Spiral wave (FIG. 14B) | 1% |

The surgical mesh samples, both with and without fixation components, were secured on commercially available polypropylene hernia mesh and were tested to evaluate adhesion/fixation strength on mammalian tissue. As seen in FIG. 15, after initial positioning of the samples, the adhesion strength, as measured in friction coefficient of the commercial polypropylene sample (Sample A) without any fixation component structure, was measured to be about 0.9 ± 0.1, polypropylene with a spiraled wave patterned fixation component with cut barb structure as seen in FIGS. 3A and 14A (Sample B) was measured to be about 1.2 ± 0.1, and polypropylene with a spiraled wave patterned fixation component with grip barb structure as seen in FIGS. 3B and 14B (Sample C) was measured to be about 1.6 ± 0.3. The highest surface resistance (lowest migration) was measured in Sample C with a friction coefficient of 1.9 indicating over 90% improvement in adhesive properties compared to base commercial polypropylene surgical mesh. Furthermore, after removing and repositing the surgical mesh, both Samples B and C, which comprise the fixation component maintained at a high friction coefficient, i.e., retained adhesive properties compared to bare Sample A.

It should be understood that various aspects disclosed herein may be combined in different combinations than the combinations specifically presented in the description and accompanying drawings. For example, features, functionality, and components from one embodiment may be combined with another embodiment and vice versa unless the context clearly indicates otherwise. Similarly, features, functionality, and components may be omitted unless the context clearly indicates otherwise. It should also be understood that, depending on the example, certain acts or events of any of the processes or methods described herein may be performed in a different sequence, may be added, merged, or left out altogether (e.g., all described acts or events may not be necessary to carry out the techniques).

Unless otherwise specifically defined herein, all terms are to be given their broadest possible interpretation including meanings implied from the specification as well as meanings understood by those skilled in the art and/or as defined in dictionaries, treatises, etc. It must also be noted that, as used in the specification and the appended claims, the singular forms "a," "an" and "the" include plural referents unless otherwise specified, and that the terms "comprises" and/ or "comprising," when used in this specification, specify the presence of stated features, elements, and/or components, but do not preclude the presence or addition of one or more other features, steps, operations, elements, components, and/or groups thereof.

Without excluding further possible embodiments, certain example embodiments are summarized in the following clauses:
Clause 1: A surgical mesh comprising: a reinforcement component; one or more fixation component comprising an elongated body with one or more surface structure on or extending out from the elongated body; and a securing component for attaching the fixation component to at least one surface of the reinforcement component.
Clause 2: The surgical mesh of clause 1, wherein the reinforcement component comprises biocompatible material, bioabsorbable materials, non-bioabsorbable materials, or any combination thereof.
Clause 3: The surgical mesh of clause 1 or clause 2, wherein the reinforcement component comprises a knit structure, woven structure, non-woven structure, or any combination thereof.
Clause 4: The surgical mesh of any one of clauses 1-3, wherein the reinforcement component comprises a plurality of pores, wherein the pores have an average diameter ranging from about 0.1 mm to about 5.0 mm.
Clause 5: The surgical mesh of any one of clauses 1-4, wherein the fixation component comprises biocompatible material, bioabsorbable materials, non-bioabsorbable materials, or combinations thereof.
Clause 6: The surgical mesh of any one of clauses 1-5, wherein the surface structure comprises a spur, an anchor, a hook, a barb, a grip, a microstructure, or any combination thereof.
Clause 7: The surgical mesh of any one of clauses 1-6, wherein the securing component comprises a sewing yarn, an adhesive, or any combination thereof.
Clause 8: The surgical mesh of any one of clauses 1-10, wherein the fixation component comprises about 0.1 % coverage to about 80 % coverage on the surface of the reinforcement component, based on a total surface area of the reinforcement component.
Clause 9: The surgical mesh of any one of clauses 1-8, wherein the fixation component is attached to the surface of the reinforcement component in a patterned configuration.
Clause 10: The surgical mesh of any one of clauses 1-9, wherein the surgical mesh is cut to a desired size and/or shape to conform to a specific shape and/or size of a target anatomy.
Clause 11: The surgical mesh of any one of clauses 1-9, wherein the surgical mesh is folded or rolled, to avoid fixation of the reinforcement component to itself.
Clause 12: The surgical mesh of any one of clauses 1-9, wherein the surgical mesh is folded or rolled to avoid fixation of the surgical mesh to a non-target anatomy.
Clause 13: A method for improving fixation of surgical mesh to a tissue, the method comprising: (a) providing a surgical mesh comprising a reinforcement component a fixation component; and (b) securing the fixation component to surface of the reinforcement component, wherein the fixation component comprises an elongated body with one or more surface structure on or extending out from the elongated body configured to grip to tissue.
Clause 14: The method of clause 13, wherein the surface structure comprises a spur, an anchor, a hook, a barb, a grip, a microstructure, or a combination thereof.
Clause 15: The method of clause 13 or clause 14, wherein the securing step further comprises sewing the fixation component to the surface of the reinforcement component with a sewing yarn.
Clause 16: The method of any one of clauses 13-15, wherein the securing step further comprises sewing the fixation component on the surface of the reinforcement component in a patterned configuration.
Clause 17: A method of treating a patient using a surgical mesh, the method comprising: (a) applying the surgical mesh of any one of clauses 1-12 to damaged tissue area to provide a contact induced adhesion between the tissue area and the surgical mesh; (b) securing the fixation component to secure the surgical mesh with the damaged tissue area; and (c) promoting tissue repair through and around the surgical mesh.
Clause 18: The method of clause 17, further comprising cutting the surgical mesh to a desired size or shape before the applying in step (a) to conform to a specific shape or size of the damaged tissue area.
Clause 19: The method of clause 17 or clause 18, wherein the applying in step (a) further comprises rolling or folding the surgical mesh and inserting the surgical mesh to the damaged tissue area.
Clause 20: The method of clause 19, wherein the securing in step (b) further comprises removal and repositioning of the surgical mesh without substantial reduction in adhesion strength.

## Claims

1. A surgical mesh (100) comprising:
a reinforcement component (110);
one or more fixation component (120) comprising an elongated body with one or more surface structure (123) on or extending out from the elongated body (121); and
a securing component (130) for attaching the fixation component to at least one surface of the reinforcement component.

2. The surgical mesh (100) of claim 1, wherein the reinforcement component (110) comprises biocompatible material, bioabsorbable materials, non-bioabsorbable materials, or any combinations thereof.

3. The surgical mesh (100) of claim 1 or claim 2, wherein the reinforcement component (110) comprises a knit structure, woven structure, non-woven structure, or any combination thereof.

4. The surgical mesh (100) of any of claims 1-3, wherein the reinforcement component (110) comprises a plurality of pores (114), wherein the pores have an average diameter ranging from about 0.1 mm to about 5.0 mm.

5. The surgical mesh (100) of any of claims 1-4, wherein the fixation component (120) comprises biocompatible material, bioabsorbable materials, non-bioabsorbable materials, or combinations thereof.

6. The surgical mesh (100) of any of claims 1-5, wherein the surface structure (123) comprises a spur, an anchor, a hook, a barb, a grip, a microstructure, or any combination thereof.

7. The surgical mesh (100) of any of claims 1-6, wherein the securing component (130) comprises a yarn, an adhesive, or any combination thereof.

8. The surgical mesh (100) of any of claims 1-7, wherein the fixation component (120) is attached to the surface of the reinforcement component (110) in a patterned configuration, wherein the fixation component comprises about 0.1 % coverage to about 80 % coverage on the surface of the reinforcement component, based on a total surface area of the reinforcement component.

9. The surgical mesh (100) of any of claims 1-8, wherein the surgical mesh is cut to a desired size and/or shape to conform to a specific shape and/or size of a target anatomy.

10. The surgical mesh (100) of any of claims 1-9, wherein the surgical mesh is folded or rolled to avoid fixation of the reinforcement component (110) to itself.

11. The surgical mesh (100) of any of claims 1-9, wherein the surgical mesh is folded or to avoid fixation of the surgical mesh to a non-target anatomy.

12. A method for improving fixation of surgical mesh to a tissue, the method comprising:
(a) providing a surgical mesh (100) comprising a reinforcement component (110) and at least one fixation component (120); and
(b) securing the at least one fixation component to a surface of the reinforcement component;
wherein the at least one fixation component comprises an elongated body with one or more surface structure (123) on or extending out from the elongated body configured to grip to tissue.

13. The method of claim 12, wherein the surface structure (123) comprises a spur, an anchor, a hook, a barb, a microstructure, or a combination thereof.

14. The method of claim 12 or 13, wherein the securing step further comprises sewing the fixation component (120) to the surface of the reinforcement component (110) with a yarn.

15. The method of any of claims 12-14, wherein the securing step comprises sewing the fixation component (120) on the surface of the reinforcement component (110) in a patterned configuration.
